Europäisches Patentamt

European Patent Office

Office européen des brevets

(19)

(11) Publication number: **0 258 186 B1**

(12) **EUROPEAN PATENT SPECIFICATION**

(45) Date of publication of patent specification: 22.05.91 (51) Int. Cl.5: **A61K 7/30**

(21) Application number: 87810464.5

(22) Date of filing: 17.08.87

(54) Enzyme containing denture cleansers and method of use.

(30) Priority: 28.08.86 US 901105

(43) Date of publication of application:
02.03.88 Bulletin 88/09

(45) Publication of the grant of the patent:
22.05.91 Bulletin 91/21

(84) Designated Contracting States:
AT BE CH DE FR GB IT LI LU NL SE

(56) References cited:
FR-A- 2 520 614
GB-A- 1 391 318
GB-A- 1 527 010
SU-A- 1 082 431

CHEMICAL ABSTRACTS, vol. 98, no. 4, January 1983, page 325, abstract no. 22089m, Columbus, Ohio, US; & JP-A-57 142 910 (SANKIN INDUSTRY CO. LTD) 03-09-1982

(73) Proprietor: WARNER-LAMBERT COMPANY
201 Tabor Road
Morris Plains New Jersey 07950(US)

(72) Inventor: Gallopo, Andrew R.
133 Wessington Avenue
Garfield New Jersey 07026(US)

(74) Representative: Jones, Michael Raymond
HASELTINE LAKE & CO. Hazlitt House 28
Southampton Buildings Chancery Lane
London WC2A 1AT(GB)

EP 0 258 186 B1

## Description

This invention relates to denture cleanser tablets and more particularly to an improved water-soluble denture cleanser tablet containing enzymatic cleansing agents.

Dentures may be cleansed wither by immersion in a cleansing solution or by brushing with a cleansing agent in the manner of natural teeth. The former method is generally preferred, partly for convenience but primarily since brushing tends to mar the smooth surfaces of plastic dentures.

Cleansing solution may be preformed liquid solutions but for storage convenience are usually in solid form in which case the cleansing solution is prepared at the time of use by dissolving the solid form in tap water. The solid form cleanser may be in the form of loose powder or granules or may be in the form of tablets. The tablet form is the preferred form.

In addition, denture cleanser tablets usually also contain one or more active oxygen compounds such as sodium perborate monohydrate, potassium peroxydisulphate, potassium monopersulphate, sodium carbonate peroxide, potassium peroxydiphosphate, diperisophthalic acid, monoperphthalic acid and the like, which cause the tablets to evolve micro-bubbles of active or nascent oxygen as they are dissolved in water and provide an oxidizing cleansing action including a bleaching effect on the denture stains. Generally, they also contain a surfactant to lower the surface tension and to enhance the cleansing action.

Further, denture cleanser tablets usually also contain various other relatively inactive ingredients such as fillers, extenders, binders, indicators or dyes and flavours and the like. Lubricant systems, such as magnesium stearate or talc, to facilitate smooth and even flow of the dry granular materials of the formulations during tableting operations may also be added.

In the past, improvement in denture cleansers has been carried out primarily by manipulating the oxidizing system, e.g., perborate and persulphate, and pH in order to achieve improved food stain removal. Hypochlorite generating formulations are well known to achieve a high level of plaque and moderate level of food stain removal, but it is known that they are harmful to denture metals and generate an odour unacceptable to the consumer.

Food stain does not adhere directly to denture materials but to biological accretions, such as denture plaque. Denture plaque is a complex glycoprotein which is believed responsible, in part, for denture odour. The incorporation and use of enzymes in denture cleansers is also known. food materials mucin and plaque are known to be attacked by appropriate enzymes. U.S. Patent Nos. 4,155,868 4,417,993 and 3,962,107 disclose denture cleanser tablets containing enzymes.

Although it is desirable to incorporate enzymes in denture cleanser tablets, difficulties arise because of the inactivating effect of the active oxygen when compressed with the enzyme. U.S. Patent No. 3,962,107 purports to obviate this problem by the separation of the enzymatic and active oxygen cleansing components in separate layers of the tablet. It is stated that the faster dissolving enzyme layer permits enzymatic cleansing activity to proceed before the dissolution of the active oxygen layer reaches a level which would inactivate the enzyme. On the other hand, U.S. Patent No. 4,155,868, which corresponds to GB 1527010, discloses a single layer tablet in which an enzyme, active oxygen compounds and an effervescence producing composition are allegedly incorporated in such a manner that they are retained in a usefully stable form until the tablet is ready for use. This document states that by careful control of the particle sizes, the overall water content and the surface treatments of selective components, the enzyme is not detrimentally inactivated during storage. The document further states that to provide added stability while in the dry tablet, the enzyme may be granulated or coated.

JP-A-57142910 also discloses the use of an enzyme in denture cleansing formulations. The enzymes suggested are protease, amilase, papain, lipase and dextranase.

Thus, while the art has proposed denture cleansers and particularly denture cleanser tablets which incorporate the added advantages associated with enzymes, it remains desirable to provide denture cleansers containing enzymes which are more readily and more easily manufactured and moreover which provide superior end use results, such as plaque and stain removal, than heretofore achieved.

SU-A-1082431 discloses the use in a toothpaste composition of an alkaline protease enzyme derived from Bacillus licheniformis and a potassium chloride stabilizer.

It has been surprisingly found herein that an improved water-soluble, effervescent denture cleanser, preferably in single layer tablet form, having superior plaque and stain removal properties, can be provided by utilizing certain alkaline proteolytic enzymes.

According to the present invention there is provided a stable, water soluble denture cleanser tablet comprising:

(i) an alkaline proteolytic enzyme prepared by the fermentation of Bacillus licheniformis, the enzyme having a specific activity of at least approximately 6.2 as measured at a pH of approximately 8.0, wherein

2

the specific activity is determined by allowing the enzyme to hydrolyse azocasein for 30 minutes at 40°C and precipitating undigested protein with trichloracetic acid and determining the quantity of digested products by spectrophotometry;

(ii) from 10 to 40% by weight, based on the weight of the total composition, of at least one active oxygen compound;

(iii) from 30 to 50% by weight of the total composition of an effervescence-producing composition comprising an acid and an alkali metal carbonate; and

(iv) optionally, one or more of the following ingredients: lubricants, surfactants, indicator dyes, flavours, anti-foam agents, chelating agents, fragrances, water-soluble fillers (such as, for example, sodium sulphate); wherein the total percentage of ingredients (i) - (iv) equals 100%.

The active oxygen compound need not be utilized, in which case the amount of component (iii) above may be from 30 to 90% by weight of the total composition.

Preferred enzymes falling within the definition of (i) above are described below.

Alcalase is an alkaline proteolytic enzyme available from Novo and is prepared by submerged fermentation of a selected strain of Bacillus licheniformis. The major enzyme component in Alcalase is Subtilisin Carlsberg, which is an endoproteinase of the serine type. Milezyme APG is a bacterial alkaline protease identified as E.C.3.4.21 and is available from Miles Laboratories, Inc. Milezyme APG is produced by the controlled fermentation of Bacillus licheniformis and is an endoproteinase capable of hydrolyzing the interior peptide bonds of protein molecules. It has been surprisingly discovered herein that the utilization of Alcalase or Milezyme APG in a water-soluble denture cleanser formulation provides superior plaque removal and in combination with an active oxygen compound provides superior plaque removal and stain removal.

The present invention is a water-soluble denture cleanser in tablet form comprising an enzyme and at least one active oxygen compound in an effervescent composition in which each of the cleansing functions, namely the enzymatic, the oxidizing and the mechanical cleansing actions are effective.

The denture cleanser tablets of the present invention may be in single layered or multi-layered form. In the case of single layered tablets, which are preferred herein, the enzyme is incorporated in the tablet in a granular encapsulated form. As such, inactivation of the enzyme by contact with the active oxygen compound is obviated. In multi-layered tablet form, the enzyme may be utilized in an uncoated or non-encapsulated state.

The enzyme component of the denture tablets according to the invention is at least one alkaline proteolytic enzyme as defined in i) above, which is capable of acting on food, food degradation products, mucin and plaque and is preferably selected from Alcalase and Milezyme APG. Milezyme APG is preferred. In furtherance of the present invention, the proteolytic enzyme is preferably employed in a granulated, encapsulated form.

When utilized in the denture cleanser compositions of the present invention the active oxygen component, e.g. peroxygen compound, is a compund which forms active oxygen, e.g. hydrogen peroxide, when placed in solution. Typical active oxygen compounds include sodium perborate monohydrate and tetrahydrate, anhydrous sodium perborate, potassium persulphate, sodium carbonate peroxide, diperisophthalic acid, monoperphthalic acid, potassium peroxydiphosphate, sodium aluminium amino- hydroperoxide and the like. The peroxygen or active oxygen component is preferably employed in a granular form and in an amount of from 10 to 40% weight, preferably from 15 to 25% based on the total composition and most preferably 20-23%.

The effervescence producing composition employed in the tablets herein is a carbon dioxide generating mixture comprising a carbonate compound and an acid. By "carbonate compound" is meant an alkali metal carbonate or bicarbonate, such as for example carbonate or bicarbonate of sodium or potassium. The acid component is preferably citric or tartaric acid or mixtures thereof but may be other water soluble acids including mixtures thereof such as, for example, sodium or potassium acid phosphates, gluconic acid, malic acid etc. The acid and the carbonate compounds are generally employed in approximately stoichiometric amounts although a very slight excess of acid may be employed to effectively generate the potential carbon dioxide. Large excesses of acid are generally not desirable especially if the enzyme has an optimum pH in the neutral to slightly alkaline range.

One component which has no cleansing function but is generally employed in tablet preparation is a lubricant. For this purpose representative water-soluble lubricants include magnesium stearate, sodium benzoate, and polytetrafluoroethylene. A minor amount of surfactant may also be included if desired. Suitable surfactants include anionic water-soluble salts of organic sulphoxy compounds having in their molecular structure an alkyl or acyl radical of carbon content within the range of about 8 to 18. Representative groups are alkali metal alkyl sulphates, sulphoacetates and benzenesulphonates. A preferred surfactant is sodium lauryl sulphoacetate.

In addition, other ingredients may also be incorporated. Generally, an indicator dye and a flavour ingredient are included. An anti-foam agent may also be added. Since a completely water-soluble tablet is contemplated, insoluble fillers and nonessential ingredients other than the foregoing are avoided. Suitable dyes for inclusion in the tablets are those which are readily oxidized by the peroxygen compound in about fifteen to thirty minutes and may be one having cyclohexadienimine nucleus and available as FD&C Green #1 (Guinea Green B), FD&C Blue#2, or other dyes including azo dyes. Suitable flavouring include spray dried powders of peppermint and others flavour oils conventionally employed in dental preparations. All components, essential or optional, are employed in a purity appropriate for pharmaceutical use and are readily available commercially.

The amounts of the active components will vary depending on the particular enzyme, active oxygen compound, acid and carbonate compound. When broadly expressed as weight of component per total weight of tablet, these may be found to lie within the following ranges: the enzyme up to 11.3%, eg. 6%, and from 0.001 to 2 percent, preferably 0.1 to 1% in tablets with active oxygen compounds and from 0.01 to 25%, preferably from 3 to 12% in tablets without active oxygen compounds; the active oxygen compounds from 10 to 40 percent; the effervescence producing composition comprising an acid and an alkali metal carbonate compound from 30 to 50 percent. The active oxygen component may be supplied as a single active oxygen compound or as a mixture (usually two or three) of active oxygen compounds.

The amount of acid to be included in the effervescence producing composition should be selected with consideration of the pH of the solution resulting when the tablet is dissolved in water. Failure to observe this consideration may result in premature deactivation of the enzyme. The formulations are preferably adjusted so that on dissolution of the tablet, the pH is in the range for enzyme activity of the particular enzyme used. In the case of the prefered alkaline proteases, the optimum pH range is from 7 to pH 8.5.

The other components when present may be employed in amounts hereinafter designated.

The amount of water-soluble lubricant employed may be up to 1 percent, preferably, in the range of from 0.7 to 1 percent by weight of the tablet.

Surfactants when added to improve wetting properties usually do not constitute more than approximately 1 percent of the total weight of tablet. Preferably, small amounts are added, usually in the range of from 0.4 to 1 percent.

Spray dried powder flavouring, when employed, may be added in amounts of from 0.5 to 1 percent. Dyes or colouring, if employed, are usually in the range of from 0.1 to 0.2 percent. A water-soluble dye system may be usefully employed as a timing indicator of cleaning accomplished.

The effervescent, enzyme and active oxygen containing denture cleanser tablets prepared as described are employed to clean dentures by placing in water with the denture to be cleaned for a time sufficient to effect the desired cleaning. The tablets may be from 2 to 3.2 grams in total weight and are usually employed with warm water, preferably initially 40° to 50° C in an amount sufficient to completely cover the denture. When so employed, effective and desired cleaning may be achieved in 15 to 30 minutes but in more highly stained dentures, longer periods may be desirable. The tablets when thus employed are found to effectively remove mucin, plaque, berry and grape stains which are not as readily removed by tablets not containing enzymes utilized herein. The tablets of the present invention also remove coffee, tea and other stains. Moreover, this is accomplished without brushing and usually without the overnight soaking necessary with the generally available tablets.

The following examples illustrate the invention but are not to be construed as limiting.

EXAMPLE 1

An effervescent denture cleansing tablet, in accordance with the present invention, is prepared having the following composition:

| Ingredient | Amount/grams/tablet |
|---|---|
| Sodium Bicarbonate USP Granular No. 5 | 0.171 |
| Dyes | 0.003 |
| Chelating Agent | 0.060 |
| Sodium Tripolyphosphate Anhydrous Granular Technical Grade | 0.159 |
| Sodium Carbonate (Soda Ash Dense) | 0.143 |
| Citric Acid Anydrous USP Fine Granular | 0.060 |
| Sodium Sulfate Anhydrous | 0.075 |
| Potassium Monopersulfate Compound | 0.611 |
| Sodium Perborate Monohydrate | 0.194 |
| Sodium Perborate Anhydrous | 0.042 |
| Fragrance | 0.015 |
| Surfactant | 0.010 |
| Lubricant | 0.025 |
| Milezyme APG (Miles Laboratories) | 0.100 |

The plaque removal properties of the above tablet composition is evaluated by placing 125 ml. of tap water adjusted to 45°C. in a beaker, suspending a test tile in the water and then adding the tablet composition. The tile is allowed to soak for 30 minutes. 92% plaque removal is obtained.

EXAMPLE 2

An effervescent denture cleansing tablet, in accordance with the present invention, is prepared having the following compositions:

5

| Ingredient | Amount/grams/tablet |
|---|---|
| Sodium Bicarbonate USP Granular No. 5 | 0.171 |
| Dyes | 0.003 |
| Chelating Agent | 0.060 |
| Sodium Tripolyphosphate Anhydrous Granular Technical Grade | 0.159 |
| Sodium Carbonate (Soda Ash Dense) | 0.143 |
| Citric Acid Anydrous USP Fine Granular | 0.060 |
| Sodium Sulfate Anhydrous | 0.075 |
| Potassium Monopersulfate Compound | 0.611 |
| Sodium Perborate Monohydrate | 0.194 |
| Sodium Perborate Anhydrous | 0.042 |
| Fragrance | 0.015 |
| Surfactant | 0.010 |
| Lubricant | 0.025 |
| Milezyme APG (Miles Laboratories) | 0.200 |

The plaque and stain removal properties of the above tablet composition is evaluated by placing 125 ml. of tap water adjusted to 45° C. in a beaker, suspending a test tile in the water and then adding the tablet composition. The tile is allowed to soak for 30 minutes. 85% plaque removal is obtained. A stain removal rating of 2.5 is obtained. (5.0 = 100% stain removal.)

EXAMPLE 3

A comparative effervescent denture cleansing tablet is prepared having the same composition as Examples 1 and 2 except that no enzyme is present.

The plaque and stain removal properties of the above tablet composition is evaluated by placing 125 ml. of tap water adjusted to 45° C. in a beaker, suspending a test tile in the water and then adding the tablet composition. The tile is allowed to soak for 30 minutes. Less than 10% plaque removal is obtained.

EXAMPLE 4

An effervescent denture cleansing tablet in accordance with the present invention which does not contain active oxygen compounds is prepared having the following composition:

## EXAMPLE 4

An effervescent denture cleansing tablet in accordance with the present invention which does not contain active oxygen compounds is prepared having the following composition:

| Ingredient | Amount/grams/tablet |
|---|---|
| Sodium Bicarbonate | 0.80 |
| Citric Acid, Anydrous | 0.26 |
| Sodium Carbonate | 0.21 |
| Sodium Sulfate | 0.48 |
| Chelating Agent | 0.035 |
| Lubricant | 0.055 |
| Surfactant | 0.005 |
| Fragrance | 0.005 |
| Milezyme APG | 0.100 |

The plaque removal properties of the above tablet composition is evaluated by placing 125 ml. of tap water adjusted to 45°C in a beaker, suspending a test tile in the water and then adding the tablet composition. The tile is allowed to soak for 30 minutes. 87% plaque removal is obtained.

Similar results as obtained in Examples 1, 2 and 4 are obtained employing Alcalase as the alkaline protease.

## EXAMPLE 5

A comparative effervescent denture cleansing tablet is prepared having the same composition as Example 5 except that no enzyme is present. The plaque and stain removal properties of the above tablet composition is evaluated by placing 125 ml. of tap water adjusted to 45°C. in a beaker, suspending a test tile in the water and then adding the tablet composition. The tile is allowed to soak for 30 minutes. Less than 10% plaque removal is obtained.

## EXAMPLE 6

PLAQUE REMOVED AS FUNCTION OF MILEZYME APG CONCENTRATION

| Milligrams of Milezyme APG 330 | Percent Plaque Removed |
|---|---|
| 400 | 96% |
| 200 | 94% |
| 100 | 87% |
| 25 | 77% |
| 10 | 28% |

7

The indicated quantity of enzyme was dissolved in 125 ml of 45°C, 0.1 M tris buffer, pH 8.0. A test tile is suspended in the solution for 30 minutes and the amount of plaque removed is determined.

All tested enzyme concentrations are effective in plaque removal.

EXAMPLE 7

SPECIFIC PROTEOLYTIC ACTIVITY OF SELECTED ENZYMES

| Enzyme | | pH | Specific Activity |
|---|---|---|---|
| Alcalase 2.0T | Lot PM8-1007 | 8.0 | 6.2 |
| Alcalase 2.0T | Lot PM8-1007 | 7.5 | 7.0 |
| Esperase 4.0T | Lot DE-001 | 8.0 | 2.4 |
| Esperase 4.0T | Lot DE-001 | 8.5 | 2.6 |
| Neutrase 1.5G | Lot PW2-0508 | 8.0 | 1.5 |
| Milezyme APG 330 | Lot 11656-28-3 | 8.0 | 8.7 |
| Novozyme 234 | Lot PPM-1503 | 5.5 | 3.2 |
| Papain | Lot 3971 | 8.0 | 3.0 |

The specific activity of various proteolytic enzymes has been determined. Enzymes derived from Bacillus licheniformis, that is Alcalase and Milezyme APG have much greater activity than other proteolytic enzymes not derived from Bacillus licheniformis.

Procedure:

The specific activity is determined by allowing the enzyme to hydrolyze azocasein for 30 minutes at 40°C. undigested protein is precipitated with trichloroacetic acid and the quantity of digested products, i.e., liberated azo dye, is determined by spectrophotometry. The specific activity is calculated by the formula:

Specific Activity = (Aur-Au)/(weight of enzyme in mg)

where

Aur = the absorbance at 390 nm of the digested solution.

Au = the absorbance at 390 nm of undigested solution.

The pH of the azocasein solution is adjusted to the optimum for each enzyme to be tested.

**Claims**

1. A stable, water soluble denture cleanser tablet comprising;

(i) an alkaline proteolytic enzyme prepared by the fermentation of Bacillus licheniformis said enzyme having a specific activity of at least approximately 6.2 as measured at a pH of approximately 8.0, wherein said specific activity is determined by allowing said enzyme to hydrolyze azocasein for 30 minutes at 40°C and precipitating undigested protein with trichloroacetic acid and determining the quantity of digested products by spectrophotomery;

(ii) from 10 to 40 % by weight, based on the weight of the total composition, of at least one acitve oxygen compound;

(iii) from 30 to 50 % by weight of the total composition of an effervescence-producing composition comprising an acid and an alkali metal carbonate, and

(iv) optionally, one or more of the following ingredients: lubricants, surfactants, indicator dyes, flavours, anti-foam agents, chelating agents, fragrances, water-soluble fillers; wherein the total percentage of ingredients (i) - (iv) equals 100 %.

2.  A tablet according to claim 1, wherein the alkaline proteolytic enzyme is the protease Milezyme APG identified as E.C.3.4.21

3.  A tablet according to claim 1 or 2 wherein the alkaline proteolytic enzyme is present in a granular encapsulated form.

4.  A tablet according to claim 1, 2 or 3, wherein the active oxygen compound is selected from sodium perborate monohydrate, sodium perborate tetrahydrate, anhydrous sodium perborate, potassium persulphate, sodium carbonate peroxide, diperisophthalic acid, potassium peroxydiphosphate, sodium aluminum aminohydroperoxide and mixtures thereof.

5.  A tablet according to any preceding claim, wherein the effervescence-producing composition is comprised of an acid selected from citric acid, tartaric acid, gluconic acid and malic acid and the alkali metal carbonate is selected from sodium bicarbonate, potassium bicarbonate, sodium carbonate and potassium carbonate.

6.  A tablet according to any preceding claim, wherein the alkaline proteolytic enzyme is present in an amount of up to 11.3%, preferably from 0.1 to 1 % by weight of the total tablet.

7.  A tablet according to any preceding claim, wherein the active oxygen compound is selected from anhydrous sodium perborate, sodium perborate monohydrate, potassium persulphate and mixtures thereof.

8.  A tablet according to any preceding claim, wherein the effervescence-producing composition is a mixture of citric acid, sodium carbonate and sodium bicarbonate.

9.  A method for cleansing dentures, the method comprising placing a water-soluble denture cleanser tablet as claimed in any one of claims 1 to 8 and a denture to be cleansed in an amount of water sufficient to completely cover the denture for a time sufficient to effect the desired cleaning.

10. A stable, water soluble denture cleanser tablet comprising;

(i) an alkaline proteolytic enzyme prepared by the fermentation of Bacillus licheniformis said enzyme having a specific activity of at least approximately 6.2 as measured at a pH of approximately 8.0, wherein said specific activity is determined by allowing said enzyme to hydrolyze azocasein for 30 minutes at 40° C and precipitating undigested protein with trichloroacetic acid and determining the quantity of digested products by spectrophotometry;

(ii) from 30 to 90% by weight of the total composition of an effervescence-producing composition comprising an acid and an alkali metal carbonate; and

(iii) optionally, one or more of the following ingredients; lubricants, surfactants, indicator dyes, flavours, anti-foam agents, chelating agents, fragrances, water-soluble fillers; wherein the total percentage of ingredients (i) - (iii) equals 100%.

## Revendications

1.  Une pastille de nettoyant d'appareils dentaires soluble dans l'eau et stable comprenant:

(i) une enzyme protéolytique alcaline préparée par la fermentation du Bacillus licheniformis, l'enzyme exerçant une activité spécifique au moins approximativement égale à 6,2 lorsqu'on la mesure à un pH d'approximativement 8,0, l'activité spécifique étant déterminée en laissant l'enzyme hydrolyser une azocaséïne pendant 30 mn à 40° C et en précipitant la protéine qui n'a pas été digérée à l'aide d'acide trichloracétique puis en déterminant la quantité de produits digérés par spectrophotométrie;

(ii) de 10 à 40% en poids par rapport au poids de la composition totale, d'au moins un dérivé oxygéné actif;

(iii) de 30 à 50% en poids de la composition totale d'une composition productrice d'effervescence comprenant un acide et un carbonate de métal alcalin; et

(iv) éventuellement, un ou plusieurs des ingrédients suivants: lubrifiants, agents tensioactifs, colorants indicateurs, agents de sapidité, agents antimousse, agents chélatants, parfums, charges solubles dans l'eau (telles que, par exemple, sulfate de sodium),

le pourcentage total des ingrédients (i) à (iv) égal 100%.

2. Une pastille selon la revendication 1, caractérisée en ce que l'enzyme protéolytique alcaline est la protéase Milezyme APG identifiée sous la référence E.C.3.4.21.

3. Une pastille selon la revendication 1 ou 2, caractérisée en ce que l'enzyme protéolytique alcaline est présente sous une forme encapsulée granulaire.

4. Une pastille selon la revendication 1, 2 ou 3, caractérisée en ce que le dérivé oxygéné actif est choisi dans le groupe formé par perborate de sodium monohydraté, perborate de sodium tétrahydraté, perborate de sodium anhydre, persulfate de potassium, peroxycarbonate de sodium, acide diperisophtalique, peroxydiphosphate de potassium, aminohydroperoxyde de sodium et d'aluminium et leurs mélanges.

5. Une pastille selon l'une quelconque des revendications précédentes, caractérisée en ce que la composition génératrice d'effervescence est constituée d'un acide choisi parmi: acide citrique, acide tartrique, acide gluconique et acide malique et le carbonate de métal alcalin est choisi parmi bicarbonate de sodium, bicarbonate de potassium, carbonate de sodium et carbonate de potassium.

6. Une pastille selon l'une quelconque des revendications précédentes caractérisée en ce que l'enzyme protéolytique alcaline est présente en quantités atteignant jusqu'à 11,3%, de préférence comprises entre 0 et 1% par rapport au poids total de la pastille.

7. Une pastille selon l'une quelconque des revendications précédentes, caractérisée en ce que le dérivé oxygéné actif est choisi parmi perborate de sodium anhydre, perborate de sodium monohydraté, persulfate de potassium et leurs mélanges.

8. Une pastille selon l'une quelconque des revendications précédentes, caractérisée en ce que la composition génératrice d'effervescence est un mélange d'acide citrique, de carbonate de sodium et de bicarbonate de sodium.

9. Un procédé de nettoyage d'appareils dentaires consistant à placer une pastille de nettoyant dentaire soluble dans l'eau, selon l'une quelconque des revendications 1 ou 8, et un appareil dentaire à nettoyer, dans une quantité d'eau suffisante pour recouvrir complètement l'appareil dentaire et pendant un temps suffisant pour produire le nettoyage convenable.

10. Une pastille de nettoyant d'appareils dentaires soluble dans l'eau stable comprenant:

(i) une enzyme protéolytique alcaline préparée par fermentation de Bacillus licheniformis, cette enzyme présentant une activité spécifique d'au moins approximativement 6,2, lorsqu'on la mesure à un pH d'approximativement 8,0, cette activité spécifique étant déterminée en laissant cette enzyme hydrolyser une azocaséine pendant 30 mn à 40°C et en précipitant la protéine qui n'a pas été digérée à l'acide trichloroacétique puis en déterminant la quantité de produits digérés par spectrophotométrie;

(ii) de 30 à 90% en poids par rapport à la composition totale d'une composition génératrice d'effervescence comprenant un acide et un carbonate de métal alcalin; et

(iii) éventuellement un ou plusieurs des ingrédients suivants: lubrifiants, agents tensioactifs, colorants indicateurs, agents de sapidité, agents antimousse, agents chélatants, parfums, charges solubles dans l'eau;

le pourcentage total des ingrédients (i) à (iii) étant égal à 100%.

**Ansprüche**

1. Stabile, wasserlösliche Gebißreinigungstablette umfassend:
   (i) ein alkalisches proteolytisches Enzym, hergestellt durch die Fermentation des Bacillus licheniformis, welches Enzym eine spezifische Aktivität von zumindest 6,2, gemessen bei einem pH von etwa 8,0, aufweist, wobei die spezifische Aktivität dadurch bestimmt wird, daß das Enzym dreißig Minuten lang Azocasein bei 40°C hydrolysiert, und unverdautes Protein mit Trichloressigsäure ausgefällt wird, und die Menge an verdauten Produkten mittels Spektralphotometrie bestimmt wird;
   (ii) von 30 bis 50 Gew.-%, basierend auf dem Gewicht der Gesamtzusammensetzung, zumindest einer aktiven Sauerstoffverbindung;
   (iii) von 30 bis 50 Gew.-%, bezogen auf die Gesamtzusammensetzung, einer Aufbrausen-bewirkenden Zusammensetzung umfassend eine Säure und ein Alkalimetallcarbonat und
   (iv) gegebenenfalls eines oder mehrere der folgenden Ingredientien: Gleitmittel, grenzflächenaktive Mittel, Indikatorfarbstoffe, Geschmacksstoffe, Anti-Schaummittel, Chelations-Mittel, Düfte, wasserlösliche Füllstoffe; worin der prozentuale Gesamtgehalt der Ingredientien (i) bis (iv) gleich 100% ist.

2. Tablette nach Anspruch 1, worin das alkalische proteolytische Enzym die als E.C.3.4.21 identifizierte Protease Milezym APG ist.

3. Tablette nach Anspruch 1 oder 2, worin das alkalische proteolytische Enzym in einer granulierten verkapselten Form anwesend ist.

4. Tablette nach Anspruch 2 oder 3, worin die aktive Sauerstoffverbindung ausgewählt ist aus Natriumperboratmonohydrat, Natriumperborat-tetrahydrat, wasserfreiem Natriumperborat, Kaliumpersulfat, Natriumcarbonatperoxid, Diperisophthalsäure, Kaliumperoxydiphosphat, Natriumaluminium-aminohydroperoxid und Mischungen davon.

5. Tablette nach irgendeinem vorhergehenden Anspruch, worin die Aufbrausen-bewirkende Zusammensetzung eine Säure ausgewählt aus Zitronensäure, Weinsäure, Glukonsäure, Apfelsäure und das Alkalimetallcarbonat ausgewählt aus Natriumbicarbonat, Kaliumbicarbonat, Natriumcarbonat und Kaliumcarbonat umfaßt.

6. Tablette nach irgendeinem vorhergehenden Anspruch, worin das alkalische proteolytische Enzym in einer Menge von bis zu 11,3%, vorzugsweise von 0,1 bis 1 Gew.-% der Gesamttablette anwesend ist.

7. Tablette nach irgendeinem vorhergehenden Anspruch, worin die aktive Sauerstoffverbindung ausgewählt ist aus wasserfreiem Natriumperborat, Natriumperboratmonohydrat, Kaliumpersulfat und Mischungen davon.

8. Tablette nach irgendeinem vorhergehenden Anspruch, worin die Aufbrausen-bewirkende Zusammensetzung eine Mischung von Zitronensäure, Natriumcarbonat und Natriumbicarbonat ist.

9. Verfahren zur Gebißreinigung, welches Verfahren das Einbringen einer wie in irgendeinem der Ansprüche 1 bis 8 beanspruchten wasserlöslichen Gebißreinigungstablette und eines zu reinigenden Gebisses in eine zur vollständigen Bedeckung des Gebisses ausreichende Menge Wasser während einer jur Bewirkung der gewünschten Reinigung ausreichenden Zeitspanne umfaßt.

10. Stabile, wasserlösliche Gebißreinigungstablette umfassend:
   (i) ein alkalisches proteolytisches Enzym, hergestellt durch Fermentation des Bacillus licheniformis, welches Enzym eine spezifische Aktivität von zumindest etwa 6,2, gemessen bei einem pH von etwa 8,0, aufweist, wobei die spezifische Aktivität dadurch bestimmt wird, daß das Enzym dreißig Minuten lang Azocasein bei 40°C hydrolysiert, und unverdautes Protein mit Trichloressigsäure ausgefällt wird, und die Menge an verdauten Produkten mittels Spektralphotometrie bestimmt wird;
   (ii) von 30 bis 90 Gew.-% der Gesamtzusammensetzung einer Aufbrausen-bewirkenden Zusammensetzung umfassend eine Säure und ein Alkalimetallcarbonat; und
   (iii) gegebenenfalls eines oder mehrere der folgenden Ingredientien: Gleitmittel, grenzflächenaktive Mittel, Indikatorfarbstoffe, Geschmacksstoffe, Anti-Schaummittel, Chelations-Mittel, Düfte, wasserlösliche Füllstoffe, worin der prozentuale Gesamtgehalt der Ingredientien (i) bis (iii) gleich 100% ist.